# FASCICULE DE BREVET EUROPEEN

(11) **EP 1 607 105 B1**
(45) Date de publication et mention de la délivrance du brevet: **10.08.2011**
(21) Numéro de dépôt: 05300486.7
(22) Date de dépôt: 16.06.2005
(51) Int. Cl.: A61B 5/103, A61K 49/00

(54) **Détermination de la tolérance vis-à-vis de l'application d'un produit sur la peau**
Toleranzbewertung beim Auftragen eines Produktes auf die Haut
Tolerance determination upon application of a product on the skin

(30) Priorité: 18.06.2004 FR 0451296
(43) Date de publication de la demande: 21.12.2005
(73) Titulaire: L'Oréal, 75008 Paris (FR)
(72) Inventeur: Bazin, Roland, 91570, BIEVRES (FR)
(74) Mandataire: Tanty, François

(56) Documents cités:
- FR-A- 2 845 005
- US-A- 5 144 957
- US-A- 5 702 035
- US-A1- 2002 182 149

## Description

La présente invention concerne les tests permettant de déterminer le niveau de tolérance d'un individu vis-à-vis de l'application d'un produit sur la peau, notamment un produit cosmétique.

Par « produit cosmétique », on entend au sens de la présente invention un produit tel que défini dans la Directive 93/35/CEE du 14 juin 1993 modifiant la Directive 76/768/CEE.

Il existe un besoin pour permettre aux consommateurs de disposer de produits exerçant une action plus forte afin d'obtenir des résultats plus rapidement et/ou plus prononcés.

Néanmoins, avec de tels produits, l'application risque davantage d'entraîner une réaction indésirable chez certains individus, notamment ceux ayant une peau particulièrement sensible.

L'invention vise à permettre à un individu, préalablement à l'application du produit, de déterminer son niveau de tolérance au produit, afin par exemple d'adapter la posologie au degré de sensibilité de sa peau ou changer de produit.

L'invention a ainsi pour objet un procédé pour déterminer le niveau de tolérance d'un individu vis-à-vis de l'application d'un produit sur la peau, ce procédé comportant les étapes définies dans la revendication 1.

Par « agent stimulant du système nerveux périphérique », on désigne un agent capable d'induire une réponse sensorielle liée à la mise en jeu de nerfs sensitifs cutanés dont les terminaisons affleurent au niveau du stratum cornéum.

L'action mécanique est une action de pelage par application puis enlèvement d'un organe adhésif ou une action d'abrasion.

L'action mécanique est avantageusement exercée avant l'application de la substance.

La substance contient de la capsaïcine, de l'acide lactique, de l'alcool ou du chlorure de sodium.

La peau est de préférence nettoyée avant l'application de la substance et avant également de l'exposer à l'action mécanique.

Une action de rinçage de la zone de test est de préférence effectuée à la fin du test.

On détermine avantageusement la posologie du produit en fonction de la réaction observée. Celle-ci peut être par exemple une démangeaison, une sensation de brûlure ou de piqûre.

Le produit peut être un produit anti-rides, un produit desquamant, contenant par exemple des α hydroxyacides et/ou des charges abrasives, un produit stimulant le renouvellement épidermique, un produit amincissant, un produit anti-acnéique, un produit dépilatoire, un produit solaire, un produit de maquillage ou de soin, cette liste n'étant pas exhaustive.

La substance est appliquée de préférence sur une zone sensible, par exemple sur le visage, notamment sur le sillon nasogénien.

L'invention a encore pour objet, selon un autre de ses aspects, un kit permettant de déterminer le niveau de tolérance d'un individu vis-à-vis de l'application d'un produit sur la peau comme définit dans la revendication 7. :

L'organe permettant d'exercer une fonction mécanique sur la peau peut être adhésif ou abrasif.

Le kit peut comporter en outre au moins une information utile pour prescrire un produit en fonction d'une réaction observée suite à l'application de la substance.

La substance peut être constituée par le même produit que celui qui est destiné à être appliqué sur la peau suite à l'évaluation du niveau de tolérance de celle-ci. La substance peut encore être différente du produit qui sera appliqué sur la peau suite à l'évaluation du niveau de tolérance de celle-ci, et contenir par exemple de la capsaïcine ou de l'acide lactique.

La substance peut être contenue, le cas échéant, dans un conditionnement monodose.

Le kit peut comporter une lotion de rinçage de la zone de test.

Le kit peut comporter en outre le produit destiné à être appliqué sur la peau, notamment en cas d'évaluation positive de la tolérance de celle-ci audit produit.

Un procédé de prescription d'un produit cosmétique est aussi décrit, comportant l'évaluation du degré de tolérance de la peau en mettant en oeuvre le procédé défini plus haut plus la prescription d'un produit et/ou d'une posologie en fonction du résultat de l'évaluation.

La prescription peut s'effectuer sur un lieu de vente, par exemple dans un grand magasin, un institut de beauté ou une officine, ou à distance, le consommateur pouvant recevoir la prescription par un courrier électronique ou physique, accompagnée dans ce dernier cas par le produit prescrit, le cas échéant.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique un kit conforme à l'invention,
- la figure 2 représente une variante d'organe permettant d'exercer une action mécanique sur la peau,
- la figure 3 est un schéma en blocs illustrant différentes étapes d'un procédé selon l'invention,
- la figure 4 est un exemple de table permettant d'évaluer la posologie en fonction des réactions observées, et
- la figure 5 représente de manière schématique une variante de réalisation d'un conditionnement monodose.

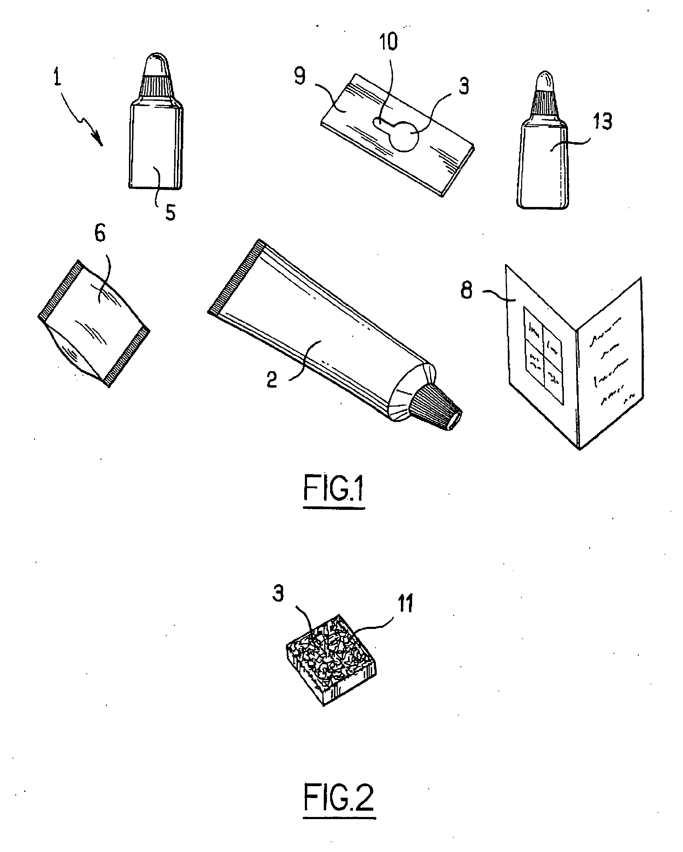

Le kit 1 représenté à la figure 1 vise à permettre d'évaluer le niveau de tolérance d'un individu vis-à-vis de l'application d'un produit sur la peau, ce produit pouvant être ou non contenu dans le kit.

Dans l'exemple considéré, le produit destiné à être appliqué sur la peau est compris dans le kit et conditionné par exemple dans un tube 2, mais il est bien évident que ce produit pourrait être conditionné autrement, par exemple dans un pot, un flacon, un récipient muni d'un applicateur ou sous la forme d'un pain solide, cette liste n'étant pas limitative. Le type de conditionnement sera choisi en fonction de la nature du produit et de la région sur laquelle le produit doit être appliqué.

Le kit 1 comporte, conformément à l'invention, un organe 3 permettant d'exercer une action mécanique sur la peau, cette action mécanique visant à diminuer sa fonction barrière.

Le kit 1 comporte en outre une substance de test dont l'application sur la peau est susceptible de conduire à une stimulation du système nerveux périphérique.

Cette substance est par exemple le produit lui-même mais elle peut être différente du produit lui-même et notamment contenir au moins un agent stimulant du système nerveux périphérique, par exemple de la capsaïcine, de l'acide lactique, de l'alcool, du chlorure de sodium.

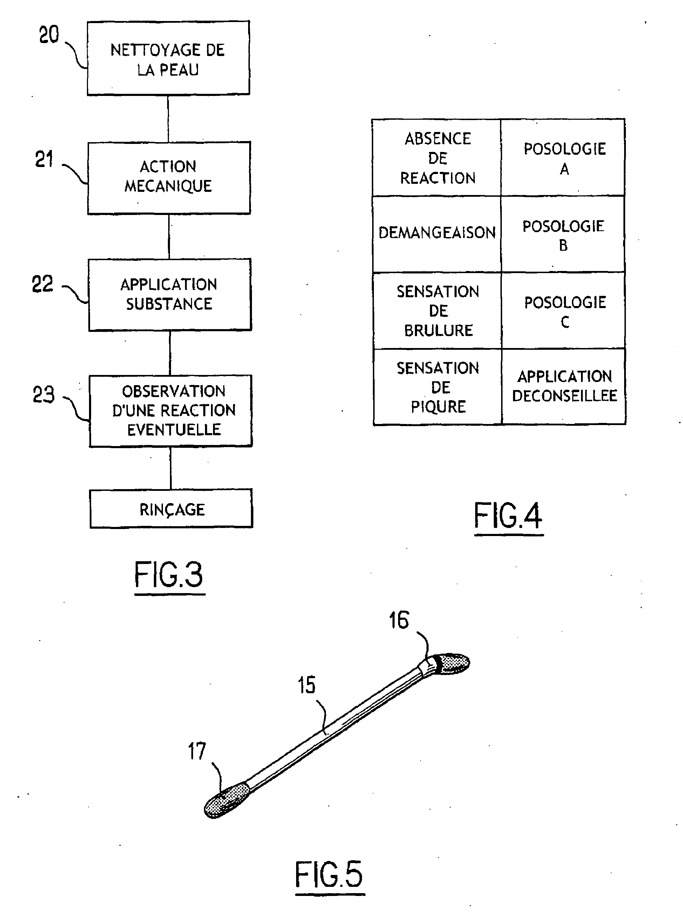

Dans l'exemple considéré, cette substance est contenue dans un flacon monodose 5, mais il est bien évident que la substance pourrait être contenue dans tout autre conditionnement, par exemple un tube genre coton-tige pourvu d'un bouchon de liquide comme décrit dans la demande FR 2 845 005 ou commercialisé par la société SWABPLUS®. Un tube 15 commercialisé par la société SWABPLUS® a été représenté schématiquement à la figure 5. Ce tube 15 comporte une extrémité sécable 16 (représentée après cassure) et une extrémité 17 destinée à l'application de la substance contenue dans le tube.

Le kit 1 comporte encore, dans l'exemple considéré, un moyen de nettoyage de la peau préalablement à l'application de la substance de test, ce moyen de nettoyage étant par exemple constitué par une lingette imprégnée d'un tensioactif et contenue dans un sachet 6 fermé hermétiquement.

Enfin, dans l'exemple considéré, le kit 1 comporte aussi une notice 8 contenant des informations permettant de déterminer une posologie d'application du produit contenu dans le récipient 2 en fonction de la réaction observée suite à l'application de la substance de test.

La notice 8 peut en variante être remplacée par tout support d'information et notamment par une étiquette présente sur l'emballage, non représenté, contenant les différents éléments du kit.

Dans l'exemple considéré, l'organe 3 permettant d'exercer une action mécanique sur la peau comporte une pastille réalisée par découpage d'un film adhésif 9, cette pastille étant pourvue d'une languette de préhension 10 facilitant son détachement du reste du film 9. La pastille est par exemple du type de celles commercialisées sous la référence Cornéofix par la société Monaderm (Monaco).

D'autres organes permettant d'exercer une action mécanique sur la peau peuvent être utilisés et l'on peut notamment, comme illustré à la figure 2, utiliser un organe 3 pourvu d'une face abrasive 11.

Conformément à l'invention, le kit peut être mis en oeuvre de la manière suivante.

Tout d'abord, l'utilisateur peut procéder dans une première étape 20 au nettoyage de la région destinée à servir de test.

Il peut s'agir par exemple d'une région du visage particulièrement sensible, telle que le sillon nasogénien.

Le nettoyage peut s'effectuer au moyen de la lingette contenue dans le sachet 6.

Ensuite, une fois la zone de test nettoyée, une action mécanique peut être exercée à l'étape 21 au moyen de l'organe 3 adhésif de la figure 1 ou abrasif de la figure 2. Dans le cas d'un organe adhésif, l'abaissement de la fonction barrière de la peau résulte de l'arrachement par l'organe adhésif des cellules superficielles de la peau lors de l'application puis de l'enlèvement de l'organe adhésif. Dans le cas d'un organe abrasif, la diminution de la fonction barrière est une action d'érosion superficielle de la peau.

L'action mécanique qui est exercée facilite la pénétration de la substance à l'étape 22. L'action mécanique peut également conduire à une sensibilisation de la peau qui amplifiera la stimulation exercée par la substance de test.

Après l'application de la substance à l'étape 22, l'utilisateur procède à l'étape 23 à l'observation d'une réaction éventuelle.

Il peut y avoir absence de réaction ou une réaction sensorielle telle que par exemple une réaction de démangeaison, de brûlure ou de piqûre.

La sensation de brûlure est par exemple voisine de celle qui est ressentie en cas de brûlure thermique. La sensation de piqûre est par exemple proche de celle qui est ressentie lorsque de l'alcool est appliqué sur une coupure.

L'application de la substance de test peut également entraîner l'apparition d'une rougeur plus ou moins intense sur la zone de test.

En fonction de la réaction observée, une posologie d'application du produit contenu dans le récipient 2 peut être déterminée, par exemple au moyen d'une table de conseil telle que celle représentée à la figure 4. Une telle table peut mettre en correspondance chaque réaction observée et la posologie conseillée correspondante.

Les posologies peuvent différer entre elles par exemple par la fréquence d'application, la quantité de produit utilisée et/ou la concentration et/ou la nature des actifs.

Par exemple, en cas d'absence de réaction, une application quotidienne peut être envisagée, en cas de légère réaction une application tous les deux jours, en cas de sensation de brûlure une application hebdomadaire et en cas de sensation de piqûre l'application du produit peut être déconseillée.

Selon la réaction observée, différents types de produits peuvent être prescrits.

A la fin du test, la zone de test est de préférence rincée, par exemple en utilisant une lotion adaptée contenue dans un flacon 13 du kit 1.

Bien entendu, l'invention n'est pas limitée à l'exemple qui vient d'être décrit.

On peut notamment réaliser encore autrement l'organe destiné à exercer une action mécanique sur la peau.

Dans toute la description, y compris les revendications, l'expression « comportant un », doit être comprise comme étant synonyme de « comportant au moins un », sauf si le contraire est spécifié.

## Revendications

1. Procédé non thérapeutique pour déterminer le niveau de tolérance d'un individu vis-à-vis de l'application d'un produit cosmétique sur la peau, ce procédé comportant :
- appliquer sur une zone de test de la peau une substance qui contient au moins un agent stimulant du système nerveux périphérique, cette substance étant capable de produire une réaction représentative d'une réaction qui pourrait être observée suite à l'application dudit produit, la substance contenant de la capsaïcine, de l'acide lactique, de l'alcool ou du chlorure de sodium,
- exercer sur la zone de test de la peau préalablement, ou postérieurement à l'application de la substance de test une action mécanique permettant d'abaisser la fonction barrière de la peau vis-à-vis de la substance,
- observer l'absence de réaction ou une éventuelle réaction de l'individu suite à l'application de la substance,
l'action mécanique étant une action de pelage par application puis enlèvement d'un organe adhésif (3) ou une action d'abrasion.

2. Procédé selon l'une quelconque des revendications précédentes, **caractérisé par le fait que** l'action mécanique est exercée avant l'application de la substance.

3. Procédé selon l'une quelconque des revendications précédentes, **caractérisé par le fait que** l'on nettoie la peau avant l'application de la substance et avant de l'exposer à l'action mécanique.

4. Procédé selon l'une quelconque des revendications précédentes, **caractérisé par le fait que** la zone de test est rincée à la fin du test.

5. Procédé selon l'une quelconque des revendications précédentes, **caractérisé par le fait que** l'on détermine une posologie du produit en fonction de la réaction observée.

6. Procédé selon l'une quelconque des revendications 1 à 5, **caractérisé par le fait que** la substance de test est appliquée sur le visage, notamment sur le sillon nasogénien.

7. Kit (1) permettant de déterminer le niveau de tolérance d'un individu vis-à-vis de l'application d'un produit sur la peau, comportant :
- une substance qui contient au moins un agent stimulant du système nerveux périphérique, la substance étant différente du produit appliqué sur la peau suite à l'évaluation du niveau de tolérance de celle-ci audit produit, la substance contenant de la capsaïcine, de l'acide lactique, de l'alcool ou du chlorure de sodium,
- un organe (3) permettant d'exercer sur la peau une action mécanique permettant d'abaisser la fonction barrière de celle-ci vis-à-vis de la substance,
- un applicateur pour appliquer la substance, **caractérisé en ce que** l'organe (3) étant distinct de l'applicateur et l'organe étant adhésif ou abrasif.

8. Kit selon la revendication 7, **caractérisé par le fait que** la substance est contenue dans un conditionnement monodose (5 ; 15).

9. Kit selon l'une quelconque des revendications 7 ou 8, **caractérisé par le fait qu'**il comporte une lotion de rinçage de la zone de test.

10. Kit selon l'une quelconque des revendications 7 à 9, **caractérisé par le fait qu'**il comporte en outre le produit destiné à être appliqué sur la peau.

11. Kit selon la revendication 10, **caractérisé par le fait que** le produit est un produit anti-rides.

12. Kit selon la revendication 10, **caractérisé par le fait que** le produit est un produit desquamant.

13. Kit selon la revendication 10, **caractérisé par le fait que** le produit est un produit stimulant le renouvellement épidermique.

14. Kit selon la revendication 10, **caractérisé par le fait que** le produit est un produit amincissant.

15. Kit selon la revendication 10, **caractérisé par le fait que** le produit est un produit anti-acnéique.

16. Kit selon la revendication 10, **caractérisé par le fait que** le produit est un produit dépilatoire.

17. Kit selon la revendication 10, **caractérisé par le fait que** le produit est un produit solaire.

18. Kit selon la revendication 10, **caractérisé par le fait que** le produit est un produit de maquillage ou de soin.

19. Kit selon l'une quelconque des revendications 7 à 18, **caractérisé par le fait que** la substance est contenue dans un tube comportant une extrémité sécable (16) et une extrémité destinée à l'application de la substance.

## Claims

1. A non therapeutic method of determining an individual's level of tolerance to application of a cosmetic product to the skin, the method comprising:
· applying a substance onto a test zone of the skin, the substance containing at least one agent for stimulating the peripheral nervous system, said substance being capable of producing a reaction representative of a reaction that might be observed after application of said product, the substance containing capsaicin, lactic acid, alcohol, or sodium chloride;
· exerting a mechanical action on the test zone of the skin for reducing the barrier function of the skin relative to the substance, said action being exerted before or after application of the test substance; and
· observing the absence of reaction or any reaction of the individual as a result of applying the substance; the mechanical action being a peeling action performed by applying and then removing an adhesive member (3) or an abrasive action.

2. A method according to any preceding claim, **characterized by** the fact that the mechanical action is exerted prior to applying the substance.

3. A method according to any preceding claim, **characterized by** the fact that the skin is cleaned before applying the substance and before being exposed to the mechanical action.

4. A method according to any preceding claim, **characterized by** the fact that the test zone is rinsed at the end of the test.

5. A method according to any preceding claim, **characterized by** the fact that a dosage is determined for the product as a function of the observed reaction.

6. A method according to any one of claims 1 to 5, **characterized by** the fact that the test substance is applied to the face, in particular to the nasolabial fold.

7. A kit (1) enabling an individual's level of tolerance relative to application of a product on the skin to be determined, the kit comprising:
· a substance that contains at least one agent for stimulating the peripheral nervous system, the substance being different from the product to be applied to the skin after evaluating the skin's level of tolerance to said product, the substance containing capsaicin, lactic acid, alcohol, or sodium chloride;
· a member (3) enabling a mechanical action to be exerted on the skin suitable for lowering the barrier function thereof relative to the substance; and
. an applicator for applying the substance;
the kit being **characterized in that** the member (3) is distinct from the applicator and the member is adhesive or abrasive.

8. A kit according to claim 7, **characterized by** the fact that the substance is contained in single-application packaging (5; 15).

9. A kit according to claim 7 or claim 8, **characterized by** the fact that it includes a lotion for rinsing the test zone.

10. A kit according to any one of claims 7 to 9, **characterized by** the fact that it further includes the substance that is to be applied to the skin.

11. A kit according to claim 10, **characterized by** the fact that the product is an antiwrinkle product.

12. A kit according to claim 10, **characterized by** the fact that the product is a peeling product.

13. A kit according to claim 10, **characterized by** the fact that the product is a product for stimulating renewal of the epidermis.

14. A kit according to claim 10, **characterized by** the fact that the product is a slimming product.

15. A kit according to claim 10, **characterized by** the fact that the product is an anti-acne product.

16. A kit according to claim 10, **characterized by** the fact that the product is a depilatory product.

17. A kit according to claim 10, **characterized by** the fact that the product is a sunscreen.

18. A kit according to claim 10, **characterized by** the fact that the product is a makeup or a care product.

19. A kit according to any one of claims 7 to 18, **characterized by** the fact that the substance is contained in a tube having a break-off end (16) and an end for applying the substance.

## Patentansprüche

1. Nicht therapeutisches Verfahren zur Bestimmung des Toleranzniveaus eines Individuums gegenüber der Anwendung eines kosmetischen Produktes auf der Haut, wobei das Verfahren aufweist:
- Anwenden auf einem Testbereich von der Haut eine Substanz, welche zumindest ein für das periphere Nervensystem stimulierendes Mittel enthält, wobei diese Substanz dazu in der Lage ist, eine Reaktion hervorzurufen, die eine repräsentative Reaktion ist, welche aufgrund der Anwendung des Produktes beobachtet werden kann, wobei die Substanz Capsaicin Milchsäure, Alkohol oder Natriumchlorid enthält,
- auf den Testbereich der Haut wird vor oder nach der Applikation der Testsubstanz eine mechanische Aktion ausgeübt, die es ermöglicht, die Barrierefunktion der Haut gegenüber der Substanz zu überwinden,
- die Abwesenheit der Reaktion oder eine eventuelle Reaktion des Individuums auf die Anwendung der Substanz werden beobachtet, wobei die mechanische Aktion eine Enthaarung durch Anwendung, dann Abhebung eines adhäsiven Elements (3) oder eine Abriebbetätigung ist.

2. Verfahren nach einem der voranstehenden Ansprüche, **dadurch gekennzeichnet, dass** die mechanische Aktion nach der Applikation der Substanz durchgeführt wird.

3. Verfahren nach irgendeinem der voranstehenden Ansprüche, **dadurch gekennzeichnet, dass** man die Haut vor der Applikation der Substanz und vor der Aussetzung gegenüber der mechanischen Aktion reinigt.

4. Verfahren nach irgendeinem der voranstehenden Ansprüche, **dadurch gekennzeichnet, dass** der Testbereich am Ende des Testes gespült wird.

5. Verfahren nach irgendeinem der voranstehenden Ansprüche, **dadurch gekennzeichnet, dass** eine Posologie des Produktes in Funktion der beobachteten Reaktion bestimmt.

6. Verfahren nach irgendeinem der Anspruche 1 bis 5, **dadurch gekennzeichnet, dass** die Testsubstanz auf dem Gesicht und insbesondere auf die Nasoblabialfalte angewendet wird.

7. Kit (1) bzw. Anordnung, das es ermöglicht, das Toleranzniveau eines Individuums gegenüber der Anwendung eines Produktes auf der Haut zu bestimmen, das aufweist:
- eine Substanz, die zumindest ein Mittel enthält, das das periphere Nervensystem anregt, wobei sich die Substanz von dem Produkt unterscheidet, das auf die Haut appliziert wird, gefolgt von der Bewertung des Toleranzniveaus von dieser zu diesem Produkt, wobei die Substanz Capsaicin, Milchsäure, Alkohol oder Natriumchlorid enthält,
- ein Element (3), das es ermöglicht, auf die Haut eine mechanische Aktion auszuüben, die es ermöglicht, die Barrierefunktion von dieser gegenüber der Substanz zu überwinden,
- eine Appliziereinrichtung, um die Substanz zu applizieren, **dadurch gekennzeichnet, dass** sich das Element (3) von der Appliziereinrichtung unterscheidet und das Element adhäsiv oder abrasiv ist.

8. Kit bzw. Anordnung nach Anspruch 7, **dadurch gekennzeichnet, dass** die Substanz in einer Aufmachung einer Einzeldosis (5; 15) enthalten ist.

9. Kit bzw. Anordnung nach irgendeinem der Ansprüche 7 oder 8, **dadurch gekennzeichnet, dass** dieses eine Spüllotion für den Testbereich aufweist.

10. Kit bzw. Anordnung nach irgendeinem der Ansprüche 7 bis 9, **dadurch gekennzeichnet, dass** es außerdem das Produkt aufweist, dass dazu bestimmt ist, auf die Haut appliziert zu werden.

11. Kit bzw. Anordnung nach Anspruch 10, **dadurch gekennzeichnet, dass** das Produkt ein Produkt gegen Schuppen ist.

12. Kit bzw. Anordnung nach Anspruch 10, **dadurch gekennzeichnet, dass** das Produkt ein Produkt zum Abschuppen ist.

13. Kit bzw. Anordnung nach Anspruch 10, **dadurch gekennzeichnet, dass** das Produkt ein Produkt ist, dass die Erneuerung der Epidermis anregt.

14. Kit bzw. Anordnung nach Anspruch 10, **dadurch gekennzeichnet, dass** das Produkt ein Produkt zur Verdünnung bzw. zur Verjüngung ist.

15. Kit bzw. Anordnung nach Anspruch 10, **dadurch gekennzeichnet, dass** das Produkt ein Produkt gegen Akne ist.

16. Kit bzw. Anordnung nach Anspruch 10, **dadurch gekennzeichnet, dass** das Produkt ein depillierendes Produkt ist.

17. Kit bzw. Anordnung nach Anspruch 10, **dadurch gekennzeichnet, dass** das Produkt ein Sonnenprodukt ist.

18. Kit bzw. Anordnung nach Anspruch 10, **dadurch gekennzeichnet, dass** das Produkt ein Schmink- oder Pflegeprodukt ist.

19. Kit bzw. Anordnung nach irgendeinem der Ansprüche 7 bis 18, **dadurch, gekennzeichnet, dass** die Substanz in einer Tube enthalten ist, die ein zu trocknendes Ende (16) und ein Ende aufweist, dass dazu bestimmt ist, die Substanz zu applizieren.
